# NEUE EUROPÄISCHE PATENTSCHRIFT

(11) **EP 0 287 075 B2**
(45) Veröffentlichungstag und Bekanntmachung des Hinweises auf die Entscheidung über den Einspruch: **10.04.2002**
(45) Hinweis auf die Patenterteilung: 18.01.1995
(21) Anmeldenummer: 88105895.2
(22) Anmeldetag: 13.04.1988
(51) Int. Cl.: C12N 15/22, C12N 5/10

(54) **Verfahren zur Konstruktion einer animalen Zellinie für die Herstellung von humanem Interferon-beta**
Process for the construction of an animal cell line for the production of human beta-interferon
Procédé de construction d'une lignée cellulaire animale pour la préparation de bêta-interféron humain

(30) Priorität: 14.04.1987 DE 3712564
(43) Veröffentlichungstag der Anmeldung: 19.10.1988
(73) Patentinhaber: Dr. Rentschler Biotechnologie GmbH, 88471 Laupheim (DE)
(72) Erfinder: Reiser, Walter, Dr., D-6900 Heidelberg (DE); Joester, Karl-Eduard, Dipl.-Bioch., D-7959 Walpertshofen (DE); Wolf, Wieland, Dr., D-7959 Bronnen (DE)
(74) Vertreter: Weiss, Wolfgang, Dipl.-Chem. Dr.

(56) Entgegenhaltungen:
- EP-A- 0 163 993
- ARZNEIMITTELFORSCHUNG/DRUG RESEARCH, Band 37(I), Nr. 4, 15. April 1987, Seiten 482-485, Aulendorf, US; W. REISER et al.: "Recombinant human interferon beta from mammalian cell lines"
- MOLECULAR AND CELLULAR BIOLOGY, Band 4, Nr. 1, Januar 1984, Seiten 166-172, American Society for Microbiology, Washington, D.C., US; F. McCORMICK et al.:"Inducible expression of amplified human beta interferon genes in CHO cells"
- DNA, Band 3, Nr. 4, 1984, Seiten 297-308, Mary Ann Liebert, Inc., Publishers, New York, US; Y. CHERNAJOVSKY et al.: "Efficient constitutive production ofhuman fibroblast interferon by hamster cells transformed with the IFN-beta1 gene fused to an SV40 early promoter"
- CHEMICAL ABSTRACTS, Band 107, Nr. 3, 20. Juli 1987, Seite 190, Zusammenfassung Nr. 18859z, Columbus, Ohio, US; S. WU et al.: "Enhancing effect of the SV40 DNA HindIII B fragment on the expression of human beta-interferon gene in E. coli"
- THE JOURNAL OF BIOLOGICAL CHEMISTRY, Band 262, Nr. 30, 25. Oktober 1987, Seiten 14600-14605, The American Society of Biochemistry and Molecular Biology, Inc.,US; H.S. CONRADT et al.: "Structure of the carbohydrate moiety of human interferon-beta secreted by a recombinant chinese hamster ovary cell line"

## Beschreibung

Es wird ein Verfahren zur Herstellung von IFN-beta in einer animalen Wirtzelle beschrieben. Das in hoher Ausbeute aus dem Zellüberstand isolierte IFN-beta besitzt eine spezifische Aktivität von ca. 3.8 x 10⁸ IE/mg Protein. Biologische und immunologische Tests zeigen, daß das isolierte IFN-beta weitestgehend mit dem natürlichen IFN-beta identisch ist. Das mit dem beschriebenen Verfahren hergestellte IFN-beta ist zu 95% glykosyliert, wobei die Glykosylierung in Struktur und Sequenz weitgehend mit der von natürlichem IFN-beta übereinstimmt, ohne jedoch mit ihr identisch zu sein.

Interferone stellen eine Gruppe antiviral wirksamer Polypeptide dar, die als Folge eines Kontaktes mit exogenen Induktoren (beispielsweise Viren, Nukleinsäuren, bestimmte Antigene) von den betroffenen Zellen gebildet werden. Eine Untergruppe bilden die Beta-Interferone (IFN-beta), die hauptsächlich von Fibroblasten gebildet werden, Havell et al. (1972) und Stewart II (1979). Bislang sind 2 Spezies von beta-Interferonen bekannt, die einander aufgrund ihrer immunologischen Eigenschaften ähneln, so daß monoklonale, neutralisierende Antikörper isoliert werden konnten, die beide Interferone gleiclhermaßen inaktivieren s. Zilberstein et al. (1985). Hingegen gibt es keine Kreuzhybridisierung von IFN-beta-2 mRNA mit Proben von IFN-beta-1 cDNA in RNA-Gel-Blot-Hybridisierungsexperimenten und umgekehrt, Sehgal et al. (1980).

IFN-beta-1 aus humanen diploiden Fibroblasten (FS-4), in der Folge als IFN-beta bezeichnet, befindet sich bereits seit geraumer Zeit im klinischen Einsatz. Es wurde bereits 1983 vom Bundesgesundheitsamt für die Behandlung schwerer lebensbedrohender Virusinfektionen zugelassen. Auf Grund seiner Wirksamkeit und wegen des Mangels an anderen breit wirksamen Virostatika hat es sich in vielen Fällen als Mittel der Wahl erwiesen.

Da jedoch die Herstellung auf der Basis normaler, nichttransformierter diploider Fibroblasten den Einsatz rarer und teurer Rohstoffe sowie die Verwendung kostenintensiver Zellsubstrate erfordert und da der Rationalisierung des Verfahrensablaufs enge Grenzen gesetzt sind, schränkt der hohe Preis des Medikaments den klinischen Einsatz stark ein.

Diese Situation hat schon sehr früh die Suche nach alternativen Herstellungsmethoden stimuliert und war damit einer der wichtigsten Antriebe für die Entwicklung moderner gentechnologischer Verfahren überhaupt. Die Einschleusung des Gens für das humane IFN-beta in heterologe Wirtszellsysteme lag als einzige echte Alternative zu klassischen Optimierungsverfahren nahe, da deren Erfolgsaussichten aufgrund physiologischer Barrieren enge Grenzen gesetzt zu sein scheinen.

Von den drei prinzipiell zur Verfügung stehenden Gruppen von Wirtszellsystemen, Prokaryoten, niederen und höheren Eukaryoten wurde zunächst auf Grund des Standes der Technik sowie der extrem niedrigen Herstellungskosten allgemein dem bakteriellen Wirtssystem Escherichia coli (E.coli) der Vorzug gegeben.

Trotz der hohen Erwartungen, die in dieses Produktionssystem gesetzt wurden, ist es bisher nicht gelungen, die großen Mengen Roh-IFN-beta, die sich mit dieser Methodik herstellen lassen, im technischen Maßstab aufzuarbeiten, Taniguchi et al.(1980) und Goeddel (1980). Die Hauptursache für diese Schwierigkeiten liegt darin, daß das Rohmaterial in denaturierter Form als inclusion-bodies in der Wirtszelle vorliegt. Dies ermöglicht zwar eine effiziente Trennung von Bestandteilen der Wirtszelle, aufgrund der Probleme in der weiteren Aufarbeitung (Schwerlöslichkeit, Auftreten falscher Schwefelbrückenbindungen) waren bisher jedoch die Ausbeuten an klinisch einsetzbarem Material sehr gering. Zu der teilweise mangelnden Wirksamkeit, die vermutlich auf Konformationsänderungen infolge Denaturierung oder nicht korrekt gebildeter intrachenarer Schwefelbrücken beruht, Lawn et al. (1981), kommt die Tatsache, daß das Produkt im Gegensatz zu der natürlich vorkommenden Form nicht glykosyliert ist.

Vor diesem Hintergrund ergab sich zunehmend die Notwendigkeit, die Entwicklung eukaryotischer Expressionssysteme voranzutreiben, die es erlauben auch heterologe Genprodukte komplexerer Struktur durch korrektes "processing" in weitgehend authentischer Form herzustellen. Systeme dieser Art sind im Laufe der Zeit von einer Reihe Autoren beschrieben worden, Reyes et al. (1982), Mitrani-Rosenbaum et al.-(1983), Smith et al. (1983), McCormick et al. (1984), Chernajovsky et al. (1984), Fukunaga et al. (1984), Page et al. (1985).

### Konstruktionsprinzip

Im folgenden wird die Konstruktion einer neuen Zellinie BIC mit Hilfe gentechnologischer Methoden beschrieben, die es erlaubt, im Vergleich zu herkömmlichen Methoden große Mengen eines mit der natürlichen Substanz weitgehend identischen, nativen Produkts zu wesentlich geringeren Kosten herzustellen.

### Wirtszellinie

Als Wirtszellinie wurde die von Urlaub et al. (1980) beschriebene DHFR⁻-Mutante der permanenten CHO-Linie (chinese hamster ovary) gewählt.

### Vektoren

### Interferon-Gen

Als Ausgangsmaterial diente der Plasmid-Vektor pBR 13, der das genomische IFN-beta Gen enthält. Ein definierter Abschnitt dieser genomischen DNA wurde nach den von Maniatis et al. (1982) beschriebenen Methoden verkürzt und mit einem bereits bekannten Vektor (pSVd2-3) rekombiniert.

### Promotor

Einen weiteren wichtigen Schritt bildete die Auswahl eines geeigneten Promotors zur Regulation der Expression. Gegenüber der Möglichkeit induzierbare Promotoren einzusetzen, Hauser et al. (1982) und Brinster et al. (1982), wurde dem von Mosthaf et al. (1985) beschriebenen starken konstitutiven Promotor von SV 40 den Vorzug gegeben, der zudem noch eine enhancer Region enthält. Die eigenen nachteiligen Erfahrungen mit induktionsabhängigen Produktionsverfahren (negative Rückkopplung, Cytotoxizität) auf der einen Seite sowie die Möglichkeiten, die andererseits permanente Fermentationsverfahren selbst für obligat adhärente Zellkulturen bieten, führten zu dieser Entscheidung. Das weiter unten detailliert beschriebene Konstrukt wurde pSVIFNAsu genannt.

### Selektion

Zur Selektion und Amplifikation in Richtung auf eine hoch exprimierende Zellinie wurde das DHFR-Gen getrennt in einem zweiten Plasmid pAdD26SV(A)-3 eingeführt. Letztere ermöglicht durch die Wechselwirkung des eigenen Adenopromotors mit der enhancer-Region des SV 40 Promotors (s.o.) die Selektion auf IFN-beta Expression. Im vorliegenden Konstrukt wurde, wie erwartet, die gemeinsam transfizierte DNA in enger Nachbarschaft integriert.

### Transfektion

Der oben beschriebene Expressionsvektor, der regulatorische Sequenzen und das Strukturgen enthielt, sowie das Selektionsplasmid, das die Defizienz der Zellinie repariert, wurden gemeinsam in einem experimentell ermittelten optimalen Mischungsverhältnis nach einer Calciumphosphat-Präzipitationsmethode transfiziert.

### Amplifikation, Klonierung und Zellbank

Interferon exprimierende Klone aus dem Transfektionsansatz wurden mit einem zuerst von Kaufman et al. (1985) beschriebenen Verfahren über mehrere Stufen der Methotrexat-Konzentration ohne Zwischenselektion auf hochproduzierende Kolonien amplifiziert, anschließend kloniert und über serielle Passagen eine ausreichende Zellzahl erhalten, die nach den üblichen Methoden der Konservierung als Stammzellbank in flüssigem Stickstoff eingelagert wurden.

### Ergebnisse

### Rohstoffproduktion

Die auf dem beschriebenen Wege erhaltene Zellinie mit der Laborbezeichnung BIC 8622 (BIC, ECACC Eingangs-Nr. 87040301) sezerniert nach Konfluenz in üblichem Zellkulturmedium (modified Eagle's MEM mit Earle's Salzen) supplementiert mit 1-5 % NCS bzw. FCS in stationärer Kultur in Multitrays (NUNC) wie im Fermenter auf Mikroträgern (Cytodex III, Pharmacia) konstitutiv zwischen 0,4 und 1,6 * 10⁹ Internationale Einheiten an IFN-beta pro Tag in einem Liter Kulturüberstand.

### Anreicherung

Die Anreicherung erfolgt über Adsorption an einen Sulfopropyl-Kationenaustauscher und anschließende Immunsorption an Anti-IFN-beta-Sepharose (Celltech, Slough). Da diese Immunsorptionsmatrix monoklonale Antikörper gegen natives Fibroblasten-Interferon enthält, ist die Adsorption und Desorption unter den vom Hersteller für die Immunbindungsreaktion optimierten Bedingungen ein Hinweis auf die Identität des synthetisierten Moleküls, der mit Hilfe eines unter Benutzung der gleichen Antikörper entwickelten ELISA's quantifiziert werden konnte. Als weiterer Reinigungsschritt schließt sich eine FPLC-Gelfiltration zur Entfernung von Begleitstoffen niederen und höheren Molekulargewichts an.

### Charakterisierung

### Biologische Wirkung:

Der für den Nachweis von natürlichem IFN-beta aus FS-4 Zellen entwickelte Bioassay, der die antivirale Aktivität ausnutzt, indem er die Hemmung des cytopathischen Effekts von murinem Encephalomyocarditis-Virus (EMCV) auf eine Indikatorzellinie (FS-4) quantifiziert, modifiziert nach Havell et al. (1972), ist ohne Einschränkung auch für den Nachweis des IFN-beta aus BIC-Zellen verwendbar. Die mit diesem Test und der Proteinbestimmung nach Lowry et al. (1951) ermittelte spezifische Aktivität entspricht mit 2-3 * 10⁸ IE im Rahmen der Meßgenauigkeit den für FS-4 Interferon ermittelten Daten.

### Immunologische Charakterisierung:

Neben der Immunaftinitätschromatographie, die zur Anreicherung des IFN-beta aus BIC diente, wurde mit Hilfe eines neu entwickelten ELISA und mittels Immunblotting-Techniken der Nachweis weitgehender Übereinstimmung in den molekularen Eigenschaften des natürlichen und des rekombinanten IFN-beta geführt. Bei den verwendeten Antikörpern handelt es sich um monoklonale aus Maus Hybridomen (MAK B0-2, Celltech) und polyklonale IgG von der Ziege (Rega Institut, Leuwen).

### Proteinchemische Charakterisierung:

Die mit immunologischen Methoden ermittelten Daten wurden durch die Aminosäurenanalyse und die Sequenzierung von 15 N-terminalen Aminosäuren ergänzt. Dabei ließen sich keine Unterschiede zwischen natürlichem, Knight et al. (1980) und rekombinantem IFN-beta nachweisen. Die Untersuchung des Kohlenhydratanteils ergab für das rekombinante IFN-beta eine weitgehend einheitliche Glykosylierung, während das natürliche IFN-beta eine Kohlenhydratheterogenität aufweist.

### Experimenteller Teil

| Materialien: | |
|---|---|
| E.coli K12 DH 1 (DSM 4079) CHO DUK DHFR-BF (ECACC Eingangs-) Nr. 87041401) | Diese Zellinie ist durch Mutagenese und Selektion auf Abwesenheit der Dihydrofolatreduktase aus der Linie CHO-K1 (ATCC CCL 61) hervorgegangen, Urlaub et al. (1980). |
| pBR 13: (DSM 4074P) | Bei diesem Plasmid handelt es sich um ein Derivat des Klonierungsplasmids pBR 325, Bolivar et al. (1977), in dessen einzige Eco RI-Schnittstelle ein 1.83 kb langes DNA-Fragment eingesetzt ist. Dieses Fragment ist in dem Cosmid pCos IFN-beta enthalten (Gross et al., 1981), das seinerseits Teil einer Cosmid-Bank aus menschlicher Plazenta-DNA ist. |
| pSVd2-3: (DSM 4075P) | Dieses Plasmid ist ein Derivat des Klonierungsvektors pAT 153, das Sequenzen aus dem eukaryontischen DNA Virus SV 40, Fiers et al. (1978) enthält. Ausgangsplasmid zur Konstruktion der entsprechenden Vektor-Plasmide war eine Konstruktion zur Expression des Dihydrofolatreduktase-Gens der Maus, Subramani et al. (1981). Die verwendete Modifikation des Expressionsvektors pSV wurde von Herrn Dr. D. Huylebroeck hergestellt und ist im wesentlichen in der Arbeit von Fransen et al. (1985) beschrieben. |
| pAdD26SV(A)-3: (DSM 4076P) | Plasmid Derivat, das aus der Deletion eines 1.1kb langen DNA Fragments aus dem Klonierungsvektor pBR 322 hervorgegangen ist, Lusky et al. (1981). Weiterhin ist in dem Plasmid das Transkriptionsinitiations-Signal (Adenovirus major late promoter) aus dem eukaryontischen DNA Virus Adeno 2 enthalten sowie die cDNA des Dihydrofolatreduktase-Gens aus der Maus, das Polyadenylierungssignal und ein 200 bp langes Fragment mit dem Ursprung der Replikation aus SV 40, (Kaufman et al. 1982a). |

### Expressionssystem

Das verwendete Expressionssystem stützt sich auf eine Zellinie aus Eierstockzellen des chinesischen Zwerghamsters (CHO, ATCC CCL 61 CHO-K1), die durch Mutagenese defizient für das Enzym Dihydrofolat-Reduktase gemacht wurden, Urlaub et al. (1980). Durch Inkubation eines Calciumphosphat-Präzipitats von DNA mit diesen Zellen ist es möglich, in einer sehr geringen Häufigkeit die Aufnahme dieser DNA in die Zellen zu erreichen, Graham et al. (1973). Die so in die Zellen eingebrachte DNA wird meist verknüpft und in das Genom der betreffenden Zelle eingebaut. Dort wird sie ausschließlich im Zuge der zellulären DNA-Replikation oder durch im Zellgenom ablaufende Rekombinationsvorgänge vermehrt oder verändert, verhält sich also wie ein integraler natürlicher Bestandteil des zellulären Genoms. Im speziellen Fall wurde ein Expressionsplasmid zur Expression des menschlichen IFN-beta Gens und durch Anbieten in Mischung auch ein Plasmid zur Expression des cDNA Dihydrofolatreduktase Gens aus der Maus in die CHO Zellen eingebracht. Der Status des Expressionsplasmids läßt sich mit Hilfe der sog. Southern Blot-Technik feststellen. Dazu wird die zelluläre DNA mit Restriktionsendonukleasen gespalten und auf einem Agarosegel in einem elektrischen Feld aufgetrennt. Die DNA Spaltstücke werden dann an eine Nylon-Filtermembran gebunden und durch DNA-DNA Hybridisierung mit radioaktiv markierter Plasmid DNA detektiert.

### Isolierung des Interferon-beta-Gens

Als Ausgangsplasmid zur Isolierung von DNA-Restriktionsfragmenten mit dem IFN-beta Gen diente das Plasmid pBR 13. Zur Herstellung dieses Plasmids, wurde ursprünglich die für IFN-beta codierende mRNA aus FS 4-Fibroblasten in cDNA übersetzt und durch Hybridisierung mit komplementärer genomischer DNA ein das IFN-beta-Gen enthaltendes Cosmid pCOSIFN-beta aus einer Genbank (Humanplazenta) isoliert. Durch Spaltung des Plasmids pBR 13 mit der Restriktionsendonuklease Eco RI wurde zunächst das Fragment mit dem IFN-beta Gen aus pCosIFNbeta freigesetzt und anschließend nach Isolierung desselben durch weitere Spaltung mit den Restriktionsendonukleasen Hinc II, bzw. Nco I und Hind III kleinere DNA-Stücke mit dem IFN-beta Gen gewonnen.

### Konstruktion der Expressionsplasmide

Für die Konstruktion des Plasmids zur Expression von menschlichen IFN-beta wurde ein Expressionsvektor mit der Bezeichnung pSVd2-3 verwendet. Die funktionalen Komponenten dieses Vektors gewährleisten eine autonome DNA Replikation in E. coli sowie die Selektion der E. coli Zellen mittels Ampicillin (Ursprung der Replikation des Transfer-negativen Plasmids pAT 153 und des β-Lactamase Gens). Die Komponenten, die für eine Funktion in eukaryontischen Zellen bestimmt sind, bestehen aus der Promotor-Enhancer Region der frühen Gene des Virus SV 40 zur Initiation der Transkription der nachgeschalteten Gene sowie der des Polyadenylierungssignals des SV 40 Virus zum Anhängen einer polyA Sequenz an die transkribierte mRNA, falls dem eingesetzten Gen eine solche Signalsequenz fehlen sollte. Zwischen dem Signal zur Transkriptionsinitiation und der Polyadenylierungssequenz befinden sich mehrere synthetisch erzeugte Erkennungs- und Spaltsequenzen für Restriktionsendonukleasen zum Einsetzen von zu exprimierender DNA. Die Spaltstelle der Nuklease Xba I wurde zur Klonierung der humanen IFN-beta DNA in diesen Vektor gewählt. Dazu wurde der Vektor mit Xba I gespalten. Aus dem Plasmid pBR 13 wurde zunächst ein Eco RI Fragment mit IFN-beta Gen und aus diesem weiter ein Ncol - Hind III Subfragment mit IFN-beta Gen isoliert. Die durch die Spaltung mit den Restriktionsendonukleasen entstandenen überhängenden Einzelstrang-DNA Enden wurden durch Inkubation mit Nukleosid-Triphosphaten und der DNA-Polymerasel aus E. coli aufgefüllt. An die so erzeugten glatten DNA Enden des Fragmentes wurden nun synthetisch hergestellte Oligonukleotide mit der Erkennungssequenz der Restriktionsendonuklease Xba I enzymatisch angehängt. Überschüssige Oligonukleotide wurden durch Inkubation mit der Nuklease Xba I enffernt und es wurden dadurch gleichzeitig überhängende Einzelstrangenden geschaffen, die zu denen der Vektor-DNA komplementär waren. Die Vektor DNA und die IFN-beta DNA wurden enzymatisch zusammengefügt und in E. coli Zellen eingebracht. Die Zellen mit IFN-beta DNA wurden durch DNA-DNA Hybridisierung identifiziert und zur Reinigung größerer Mengen Plasmid-DNA herangezogen. Diese Plasmid-DNA wurde durch Spaltung mit verschiedenen Restriktionsendonukleasen charakterisiert und jenes Plasmid zum weiteren Vorgehen ausgewählt, und als pSV IPN Nco (DSM 4077P) bezeichnet, das den Erwartungen entsprach. Das Plasmid pSV IFN Nco wurde dann partiell mit dem Enzym Xba I gespalten und die Spaltstücke gelelektrophoretisch aufgetrennt. Die dem linearen Plasmid entsprechende Bande wurde nach Anfärbung mit dem Fluoreszenzfarbstoff Ethidiumbromid und Sichtbarmachung unter UV-Licht ausgeschnitten und die DNA isoliert. Die isolierte DNA wurde dann mit der Nuklease Asu II inkubiert und danach enzymatisch zum Ring geschlossen. Aus dieser Vorgehensweise resultierte die Deletion eines Xba I - Asu II Fragmentes aus dem ursprünglichen Expressionsplasmid pSV IFN Nco. Das so hergestellte Expressionsplasmid wurde pSV IFN Asu (DSM 4078P) genannt und zur Transfektion einer Dihydrofolatreduktase defizienten CHO-Zellinie eingesetzt Zellen, die mit diesem Expressionsplasmid pSV IFN Asu transfiziert worden sind und seine DNA in das Zellgenom integriert haben, werden zur Bildung einer mRNA veranlaßt, die aus einem 60 Nukleotide langen Abschnitt von SV 40 spezifischen Sequenzen und daran angeschlossen nahezu der authentischen IFN-beta mRNA besteht. Diese mRNA wird von dem Proteinsynthese-Apparat der Zelle in ein IFN-beta Protein übersetzt, das in seinem amino-terminalen Bereich und in seiner Aminosäurezusammensetzung dem natürlichen IFN-beta Protein aus humanen Fibroblastenzellen entspricht und im Gegensatz zu dem in E. coli produzierten Proteinen glykosyliert ist.

Aus den eingesetzten DNA-Spezies und der Konstruktionsweise des Expressionsplasmids pSV IFN Asu läßt sich die Primärstruktur der mRNA ableiten, die in eukaryontischen Zellen, die dieses Plasmid in ihr Genom integriert haben, gebildet wird. In Abb. II ist die Struktur des Expressionsplasmids mit detailliertem Ausdruck der relevanten Schnittstellen wiedergegeben. Eine Übersetzung der Nukleotidsequenz mit Hilfe des genetischen Code in eine Aminosäuresequenz ergibt die Primärstruktur, des authentischen IFN-beta aus menschlichen Zellen, Tavernier et al. (1984).

### Transfektion

Das Einbringen der Expressionsvektor-DNA in die CHO-Zellinie erfolgte im wesentlichen nach einer Methode von Graham et al. (1973) beschrieben und von Wigler (1979) modifizierten Methode. Im Prinzip wird bei dieser Methode den Zellen DNA angeboten, die aus einer Lösung mit CaPO4 als Copräzipitat ausgefällt wurde. Wahrscheinlich durch Phagocytose wird diese DNA dann von den Zellen aufgenommen und über noch nicht aufgeklärte Mechanismen meist aneinandergereiht und in das zelluläre Genom integriert. Dieser Prozeß wird als Transfektion von Eukaryontenzellen mit DNA bezeichnet. Durch gemeinsame Ausfällung eines Vektors zur Expression des Enzyms Dihydrofolat-Reduktase mit dem eigentlichen Expressionsvektor können bei Verwendung von Dihydrofolat-defizienten CHO Zellen nach dem Transfektionsvorgang solche Zellen selektiert werden, die DNA aufgenommen und in ihr Genom integriert haben. Dies geschieht durch Kultivierung der Zellen in einem Zellkultur-Medium, dem Bausteine für die Nukleinsäuresynthese fehlen.

### Amplifikation und Selektion

Die Kulturüberstände der so transfizierten und selektierten Zellen wurden auf beta-Interferon-Aktivität geprüft. Die in diesem Test positiven Klone wurden einem Verfahren unterworfen, das zum Ziel hatte, die in den Zellen eingebrachten DNA selektiv zu vermehren und durch Erhöhung der Kopienzahl der Interferon Gene eine Steigerung der beta-Interferon-Produktion zu erreichen. Dies ist grundsätzlich nach dem Gen-Dosis-Effekt möglich. Das zu diesem Zweck angewandte Verfahren sieht eine Selektion der beta-Interferon produzierenden Zellen in Kulturmedium vor, das den Enzyminhibitor (+)Amethopterin enthält. Durch diesen Inhibitor wird das Enzym Dihydrofolat-Reduktase, dessen Gen durch die Transfektion in die CHO-Zellen eingebracht worden war, konzentrationsabhängig gehemmt. Durch diese Hemmung besitzen solche Zellen einen Wachstumsvorteil, die die Gen-Dosis des Dihydrofolatreduktasegens über eine Vermehrung der entsprechenden DNA erhöht haben. Da in solchen DNA-Vervielfältigungsprozessen meist größere Abschnitte einbezogen sind und die transfizierten DNA-Spezies meist benachbart in das Genom der Zellen integriert werden, wird dadurch gleichzeitig auch die Gen-Dosis des beta-Interferon Gens erhöht. Durch stufenweise Erhöhung der (+)Amethopterin-Konzentration im Kulturmedium und dazwischenliegende Selektions und Expansionsphasen wurde eine Zell-Mischung hergestellt, die beta-Interferon in großer Menge an das Kulturmedium abgibt. Aus dieser Mischung wurden durch verdünntes Aussäen der Zellen in den Kulturgefäβen und Isolierung der Zell-Kolonien mit Hilfe von Metallzylindern reine Linien von Zellen hergestellt, die ebenfalls große Mengen beta-Interferon an das Kulturmedium abgeben.

Von diesen als "BIC"-Zellen bezeichneten Klonen wurde den einschlägigen Richtlinien entsprechend eine Herstellerzellbank (= Produktionszellbank) eingerichtet und Aliquots dieser Zellen beim "Public Health Laboratory Service, European Collection of Animal Cell Cultures (ECACC)" mit der Eingangs-Nr. 87040301 hinterlegt.

### Anreicherung

Aus einer konfluenten stationären Kultur von BIC, die der Produktionszellbank entnommen wurden, wurden durch mehrfache Ernte im 24-Stunden Rhythmus 15 I Kulturüberstände mit einem durchschnittlichen Interferongehalt von 225 000 IE/ml gepoolt und gemeinsam auf einen Kationenaustauscher (Sulfopropyl) aufgetragen. In 175 ml Eluat waren 3 * 10⁹ IE enthalten, was einer Ausbeute von 83 % entspricht. Die anschließende Immunsorption an BETA RESOLUTE (R) (Celltech) ergab ein Elutionsvolumen von 112 ml, in dem mit 2,2 * 10⁹ IE noch 72 % des Interferons mit einer spezifischen Aktivität von 3.8 x 10⁸ IE/mg Protein enthalten waren. Die abschließende Gelfiltration erbrachte eine Gesamtausbeute von 48 %.

### Analytik

### Biologische und immunologische Charakaterisierung

Hochgereinigte Präparationen von rekombinaten BIC-beta-IFN und natürlichen FS 4-beta-IFN wurden auf ihren Interferongehalt im antiviralen Bioassay geprüft. Parallel dazu wurde eine Proteingehaltsbestimmung nach Lowry durchgeführt. Es ergab sich für das natürliche Material eine spezifische Aktivität von etwa 2,7-3*10⁸ Internationalen Einheiten (IE) pro Milligramm Protein und für das rekombinante Material eine Aktivität von 3 - 4 x 10⁸ IE/mg. Diese Werte entsprechen dem aus der Literatur bekannten Daten für natürliches Interferon. Aufgrund der relativ hohen Meßungenauigkeit des Bioassays kann eine präzisere Bestimmung der spez. Aktivität nach dem Stand der Technik derzeit nicht erreicht werden.

Das Ausgangsmaterial, sowie die angereicherten und hochgereinigten Fraktionen wurden neben dem antiviralen Bioassay auch einem enzymgekoppelten Immuntest (ELISA) unterworfen.

Für diesen Test wird die Plastikoberfläche von Mikrotiterplatten mit polyklonalen anti-IFN-beta-Antikörpern von der Ziege beschichtet. Anschließend wird eine Verdünnung der zu bestimmenden Probe aufgebracht. Das beta-IFN bindet nun an die immobilisierten Antikörper. In dem nächsten Schritt läßt man diesen Komplex mit einer Lösung monoklonaler anti-IFN-beta-Antikörper von der Maus reagieren. Der jetzt entstandene Gesamtkomplex wird mit anti-Maus-IgG-Antikörpern, die vorher mit Meerrettich-Peroxidase konjugiert wurden, inkubiert. Nach Entfernung der ungebundenen Antikörper kann durch eine Farbreaktion die Menge der gebundenen Peroxidase und damit auch die Menge des gebundenen beta-IFN bestimmt werden.

Dieser Test wurde für natürliches, aus FS 4-Fibroblasten gewonnenes beta-IFN validiert. Dazu wurden Verdünnungsreihen von Proben mit bekannntem Interferongehalt zusammen mit Aliquots des Internationalen beta-IFN Standards (NIH, G-023-902-527) geprüft. Es ergab sich stets eine strenge Korrelation zwischen dem Bioassay und dem ELISA.

In gleichem Maße entsprachen auch die aus den Kulturüberständen von BIC-Zellen gewonnenen, angereicherten und hochgereinigten IFN-Präparationen im ELISA den Werten, die parallel im antiviralen Bioassay ermittelt wurden. Zur Standardisierung dienten in allen Fällen Aliquots des Internationalen Standards oder an diesem kalibrierte Präparationen. Damit konnte also gezeigt werden, daß auch die für den ELISA relevanten, antigenen Eigenschaften zwischen natürlichem und rekombinantem beta-IFN identisch sind.

### Sequenz/Aminosäurezusammensetzung

Das nach dem beschriebenen Verfahren hergestellte und gereinigte rekombinante IFN-beta wurde bezüglich seiner Aminosäurezusammensetzung und seiner Amino-terminalen Sequenz analysiert.

Die Teilsequenzierung bis zur 15. Aminosäure wurde mittels Edman-Abbau in einem automatischen Gasphasensequenator (Fa. Applied Biosystems, Typ 470 A) durchgeführt. Dabei wurden die resultierenden Phenylhydantoin-Derivate auf einer PTH-C18-Matrix getrennt und anschließend detektiert. Das Resultat stimmt mit den von Lawn et al. (1981), Ohno et al. (1981) und Derynck et al. (1980) publizierten Daten überein. Bei der Analyse der Aminosäurezusammensetzung, bei der die PTH-Aminosäuren nach der Hydrolyse der gleichen Trennmethode unterworfen wurden, konnten die von Knight et al. (1980) für FS-4 ermittelten Werte auch für das IFN-beta aus CHO bestätigt werden.

### Glykosyliering

Das nach Deglykosylierung oder nach Hemmung der Glykosylierung mit Tunicamycin erhaltene Polypeptid wies die gleichen elektrophoretischen Eigenschaften unter denaturierenden und nicht denatuierenden Bedingungen auf wie das gleichermaßen gewonnene IFN-beta aus FS-4. Die durch Glykopeptidase F freigesetzten Oligosaccharide wurden nach sequentiellem Abbau durch Exoglykosidase einer Methylierungsanalyse und FAB-Massenspektrometrie unterworfen. Dabei konnte festgestellt werden, daß 95 ± 5 % der Kohlenhydratseitenketten biantennären Komplex-Typs waren und folgende Struktur aufwiesen:

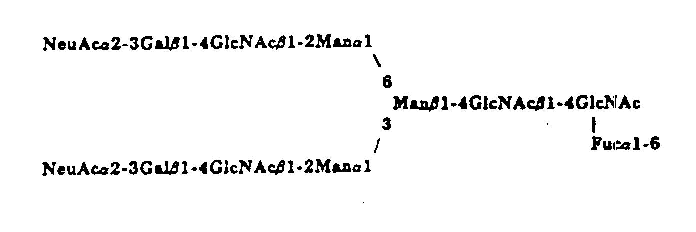

### Bibliographie:

Birnboim, H.C. (1983): A rapid alkaline extraction method for the isolation of plasmid DNA. Methods in Enzymol. 100, 243-255.

Bolivar, F. (1978): Construction and characterization of new cloning vehides. III. Derivatives of plasmid pBR 322 carrying unique Eco RI sites for selection of Eco RI generated recombinant DNA molecules. Gene 4, 121-134.

Bolivar, F., Rodriguez, R.L., Greene, P.J., Betlach, M.C., Heyneker, H.L., Boyer, H.W. (1977). Construction and characterization of new cloning vehides. II. A multipurpose cloning System. Gene 2, 95-113.

Brinster, R.L., Chen. H.Y., Warren, R., Sarthy, A., Palmiter, R.D. (1982): Regulation of metallothioneinthymidine kinase fusion plasmids injected into mouse eggs. Nature 296, 39-42.

Chernajovsky, Y., Mory, Y., Chen, L., Marks, Z., Novick, D., Rubinstein, M., Revel, M. (1984): Efficient constitutive production of human fibroblast interferon by hamster cells transformed with the IFN beta 1 gene fused to an SV 40 early promoter. DNA 3, 297-308.

Derynck, R., Content, J., Clercq, E.de, Volckaert, G., Tavernier, J., Devos, R., Fiers, W. (1980): Isolation and structure of a human fibroblast interferon gene. Nature 285, 542-547.

Dretzen, G., Bellard, M., Sassone-Corsi, P., Chambon, P. (1981): A reliable method for the recovery of DNA fragments from agarose and acrylamide gels. Anal. Biochem. 112, 295-298.

Fiers, W. Contreras, R., Haegeman, G., Rogiers, R., van der Voorde, A., van Heuverswyn, H., van Herreweghe, J., Volckaert, G., Ysebaert, M. (1978). The complete nucleotide sequence of SV 40 DNA. Nature 273, 113-120.

Flavell, R.A., Kooter, J.M., De Boer, E., Little, P.F.R., Williamson, R. 1978. Analysis of the β-delta -globin gene loci in normal and Hb Lepore DNA: Direct determination of gene linkage and intergene distance. Cell 15. 25-41.)

Fransen, L., Müller, R., Marmenout, A. Tavernier, J. van der Heyden, J., Kawashima, E., Chollet, A., Tizard, R., van Heuverswyn, H., van Vliet, A., Ruysschaert, M.R., Fiers, W. (1985): Molecular cloning of mouse tumour necrosis factor cDNA and its eukaryotic expression. Nucl. Acid. Res. 13, 4417-4429.

Fukunaga, R., Sokawa, Y., Nagata, S. (1984): Constitutive production of human interferons by mouse cells with bovine papillima virus as a vector. Proc. Natl. Acad. Sci. USA 81, 5086-5090.

Goeddel, D.V., Shephard, H.M., Yelverton, E., Leung, D., Crea, R., Sloma, A., Pestka, S. (1980): Synthesis of the human fibroblast interferon by in Escherichia coli. Nucl.Acids Res.8, 4057-4074.

Graham, F.L., van der Eb, A.J.(1973): A new technique for the assay of infectivity of human adenovirus 5 DNA. Virology 52, 456-467.

Gross, G., Mayr, U., Bruns, W., Grosveld, F., Dahl, H.H.M., Collins, J. (1981): The structure of a thirtysix kilobase region of the human chromosome including the fibroblast interferon gene IFN beta. Nucl. Acids Res. 9, 2495-2507.

Grosveld, F.G., Dahl, H.-H.M., Boer, E.de, Flavell, R.A. (1981): Isolation of beta-globin-related genes from a human cosmid library. Gene 13, 227-237.

Grunstein, M., Hogness, D. (1975): Colony hybridization: A method for the isolation of cloned DNAs that contain a specific gene. Proc. Natl. Acad. Sci. 72, 3961-3965.

Hauser, H., Gross, G., Bruns. W., Hochkeppel, H.K., Mayr, U., Collins, J. (1982): Inducibility of human beta-interferon gene in mouse L-cell clones. Nature 297, 650-654.

Havell, E.A., Vilcek, J. (1972): Production of high titered interferon in cultures of human diploid cells. Antimicrob. Agents Chemother. 2, 476-484.

Kao, F.T., Puck, T.T. (1968): Genetics of somatic mammalian cells, VII. Induction and Isolation of nutritional mutants in chinese hamster cells. Proc. Natl. Acad. Sci. USA 60, 1275-1281.

Kaufman, R.J., Sharp, P.A. (1982a): Amplification and expresssion of sequences cotransfected with a modular dihydrofolate reductase complementary DNA gene. J. Mol. Biol. 159, 601-621.

Kaufman, R.J., Sharp, P.A. (1982b): Construction of a modular dihydrofolate reductase cDNA gene: Analysis of signals utilized for efficient expression. Mol. Cell. Biol. 2, 1304-1319.

Kaufman, R.J., Sharp, P.A., Latt, S.A. (1983): Evolution of chromosomal regions containing transfected and amplified dihydrofolate reductase sequences. Mol. Cell. Biol. 3, 699-711.

Kaufman, R.J., Wasley, L.C., Spiliotes, A.J., Gossels, S.D., Latt, S.A., Larsen, G.R., Kay, R.M. (1985): Coamplification and coexpression of human tissue-type plasminogen activator and murine dihydrofolate reductase sequences in chinese hamster ovary cells. Mol. Cell. Biol. 5, 1750-1759.

Knight, E.jr., Hunkapiller, M.W., Korant, B.D., Hardy, R.W.F., Hood, L.E. (1980): Human fibroblast interferon: amino acid analysis and amino terminal amino acid sequence. Science 207, 525-526.

Laemmli, U.K. (1970): Cleavage of structural proteins during the assembly of the head of bacteriophage T4. Nature 227, 680-685.

Lawn, R.M., Adelman, J., Franke, A.E., Houck, C.M., Gross, M., Najarian, R., Goeddel, D.V. (1981): Human Fibroblast interferon gene lacks introns. Nucl. Acids Res. 9, 1045-1052.

Lowry, O.H., Rosebrough, N.J., Farr, A.L., Randall, R.J. (1951): Protein Measurement with the Folin Phenol Reagent. J. Biol. Chem. 193, 265-275.

Lusky, M., Botchan, M. (1981): Inhibition of SV 40 replication in simian cells by specific pBR-322 DNA sequences. Nature 293, 79-81.

McCormick, F., Trahey, M., Innis, M., Dieckmann, B., Ringold, G. (1984): Inducible expression of amplified human beta interferon genes in CHO cells. Mol. Cell. Biol. 4, 166-172.

Mandel, M. and Higa, A. (1970): Calcium dependent bacterio-phage DNA infection. J. Mol. Biol. 53, 159-162.

Maniatis, T., Fritsch, E.F., Sambrook, J. (1982): In: Molecular Cloning - a Laboratory Manual. Cold Spring Harbor Laboratory.

Maxam, A.M. and Gilbert, W. (1980): Sequencing end-labeled DNA with base-specific chemical cleavages. Methods in Enzymol. 65, 499-560.

Mitrani-Rosenbaum, S., Maroteaux, L., Mory, Y., Revel, M., Howley, P.M. (1983): Inducible expression of the human interferon beta 1 gene linked to a bovine papilloma virus DNA vector and maintained extrachromosomally in mouse cells. Mol. Cell. Biol. 3, 233-240.

Mosthaf, L., Pawlita, M., Gruss, P. (1985): A viral enhancer element specifically active in human haematopoietic cells. Nature 315, 597-600.

Ohno, S., Taniguchi, T. (1981): Structure of a chromosomal gene for human interferon beta. Proc. Natl. Acad. Sci. USA 78, 5305-5309.

Page, M.J. (1985): Expression of amplified human beta interferon genes using heavy metal induction in chinese hamster ovary cells. Gene 37, 139-144.

Reyes, G.R., Gavis, E.R., Buchan, A., Raj, N.B.K., Hayward, G.S., Pitha, P.M. (1982): Expression of human beta interferon cDNA under the control of a thymidine kinase promoter frorn herpes simplex virus. Nature 297, 598-601.

Rigby, P.W.J., Dieckmann,M., Rhodes, C., Berg. P. (1977): Labeling deoxyribonucleic acid to high specific activity in vitro by nick translation with DNA Polymerase I. J. Mol. Biol. 113, 237-251.

Schimke, R.T., Kaufman, R.J., Alt, F.W., Kellems, R.F. (1978): Gene Amplification and drug resistance in cultured murine cells. Science 202, 1051-1055.

Sehgal, P.B., Sagar, A.D. (1980): Heterogeneity of poly(I).poly (C)-induced human fibroblast interferon mRNA species. Nature 288, 95-97.

Shepard, H.M., Leung, D., Stebbing, N., Goeddel, D.V. (1981): A single amino acid change in IFN-beta 1 abolishes its antiviral activity. Nature 294, 563-565.

Smith, G.E., Summers, M.D., Fraser, M.J. (1983): Production of human beta interferon in insect cells infected with a baculovirus expression vector. Mol. Cell. Biol. 3, 2156-2165.

Southern E. (1980): Gel electrophoresis of restriction fragments. Methods in Enzymol. 68, 152-176.

Stewart II, W.E. (1981) In: The Interferon System, 2nd ed. Springer Verlag Wien, New York.

Subramani, S., Mulligan, R., Berg, P. (1981): Expression of the mouse dihydrofolate reductase complementary Deoxyribonucleic Acid in Simian Virus 40 vectors. Mol. Cell. Biol. 1, 854-864.

Taniguchi, T., Guarente, L., Roberts, T.M., Kimelman, D., Douhan III, J., Ptashne, M. (1980): Expression of the human fibroblast interferon gene in Escherichia coli. Proc. Natl. Acad. Sci. USA 77, 5230-5233.

Tavernier, J., Fiers, W. (1984): The presence of homologous regions between interferon sequences. Carlsberg R. 49, 359-364.

Thomas, P.S. (1980): Hybridization of denatured RNA and small DNA fragments transferred to nitrocellulose. Proc. Natl. Acad. Sci. USA 77,5201-5205.

Twigg, A.J., Sherrat, D. (1980): Trans-complementable copy-number mutants of plasmid coiel. Nature 283, 216-218.

Urlaub, G., Chasin, L.A. (1980): Isolation of chinese hamster cell mutants deficient in dihydrofolate reductase activity. Proc. Natl. Acad. Sci. USA 77, 4216-4220.

Wigler, M., Sweet, R., Sim, G.K., Wold, B., Pellicer, A., Lacy, E., Maniatis, T., Silverstein, S., Axel, R. (1979): Transformation of mammalian cells with genes from procaryotes and eukaryotes. Cell 16, 777-785.

Zilberstein, A., Ruggieri,R., Revel, M. (1985): Human interferon-beta-2: is it an interferon-inducer? In: The Interferon System, Serono Symposia 24, ed. G.B. Rossi, E. Dianzani, 73-83.

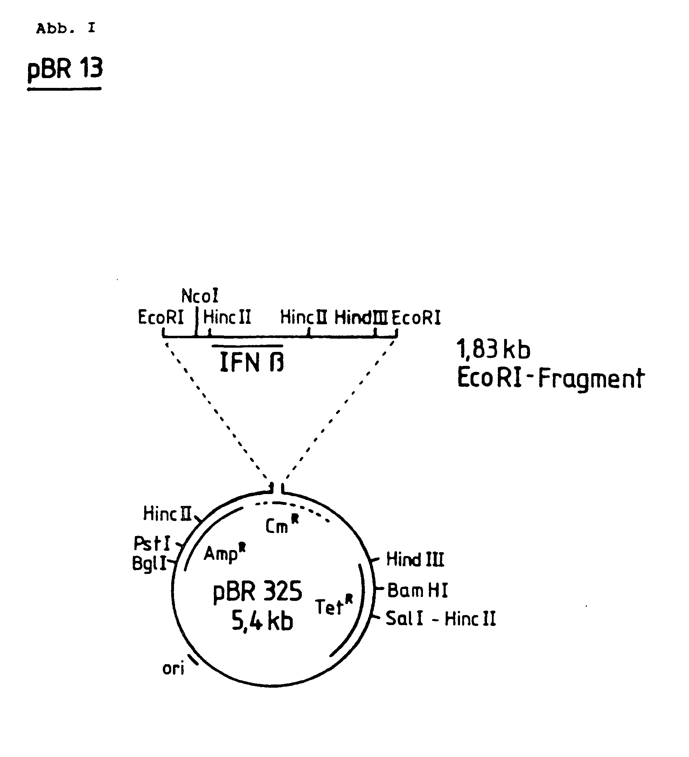

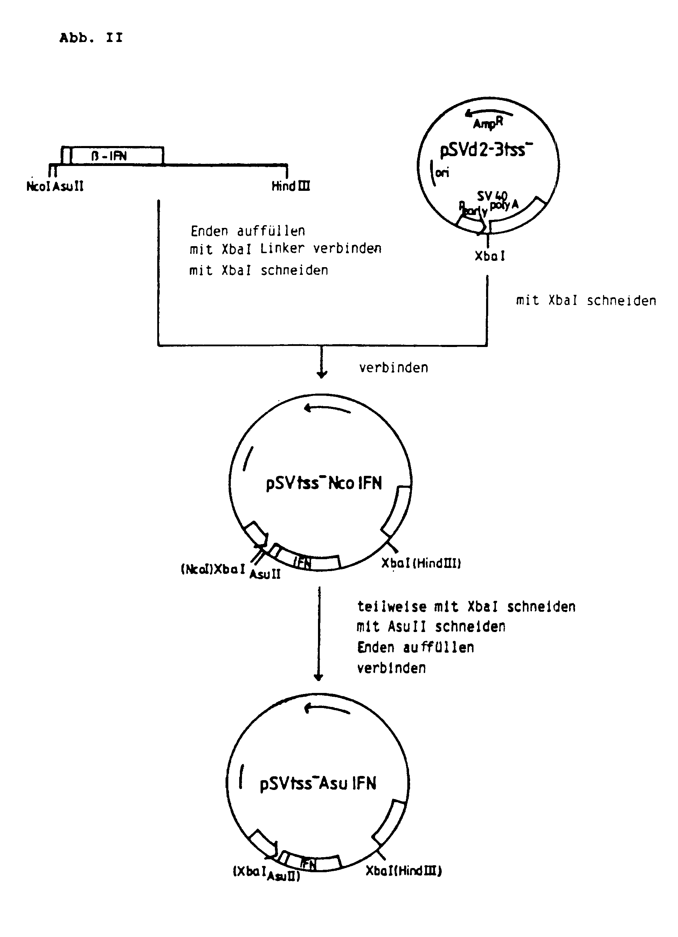

## Patentansprüche

1. Rekombinante Zelllinie BIC 8622 (ECACC 87040301).

2. Verfahren zur konstitutiven Herstellung von humanen IFN-β1, bei dem man eine rekombinante Zelllinie nach Anspruch 1 züchtet und das IFN-β1 aus dem Zellüberstand isoliert.

3. Verfahren nach Anspruch 2, weiterhin umfassend die Verwendung des erhaltenen humanen IFN-β1 zur Herstellung eines Arzneimittels.

4. Verfahren nach Anspruch 3 zur Herstellung eines Mittels für die Behandlung von Virusinfektionen.

## Claims

1. Recombinant cell line BIC 8622 (ECACC 87040301).

2. Process for the constitutive preparation of human IFN-β1, wherein a recombinant cell line according to claim 1 is cultured and the IFN-β1 is isolated from the cell supernatant.

3. Process according to claim 2, further comprising the use of the human IFN-β1 obtained for the preparation of a medicament.

4. Process according to claim 3 for the preparation of an agent for the treatment of virus infections.

## Revendications

1. Lignée cellulaire recombinante BIC 8622 (ECACC 87040301).

2. Procédé de préparation constitutive de l'IFN-β1 humaine, dans lequel on cultive une lignée cellulaire recombinante selon la revendication 1 et on isole l'IFN-β1 du surnageant cellulaire.

3. Procédé selon la revendication 2, comprenant en outre l'utilisation de l'IFN-β1 humain obtenu pour la préparation d'un médicament.

4. Procédé selon la revendication 3 pour préparer un produit pour le traitement des infections virales.
